Europäisches Patentamt

European Patent Office

Office européen des brevets

(11) Publication number: **0 257 721 B1**

# EUROPEAN PATENT SPECIFICATION

(45) Date of publication of patent specification: **15.01.92**

(51) Int. Cl.⁵: **A61K 39/39,** A61K 39/12, A61K 39/125, A61K 39/13, A61K 39/29

(21) Application number: **87201620.9**

(22) Date of filing: **27.08.87**

(54) Vaccine.

(30) Priority: **27.08.86 US 900739**

(43) Date of publication of application:
**02.03.88 Bulletin 88/09**

(45) Publication of the grant of the patent:
**15.01.92 Bulletin 92/03**

(84) Designated Contracting States:
**AT BE CH DE ES FR GB GR IT LI LU NL SE**

(56) References cited:

**JOURNAL OF VIROLOGY, vol. 57, no. 1, January 1986; B.SHERRY et al., pp. 246-257**

**DEVELOPMENTS IN BIOLOGICAL STANDARDIZATION, vol. 55, 1984, Basel (CH); A.L.VAN WEZEL et al., pp. 209-215**

**NATURE, vol. 304, no. 592825, 31 August 1983; E.A.EMINI et al., pp. 699-703**

(73) Proprietor: **DE STAAT DER NEDERLANDEN VERTEGENWOORDIGD DOOR DE MINISTER VAN WELZIJN, VOLKSGEZONDHEID EN CULTUUR**
**P.O. Box 439**
**NL-2260 AK Leidschendam(NL)**

(72) Inventor: **Van Wezel, Antonius Ludovicus**

**deceased(NL)**
Inventor: **Hazendonk, Antonius Gerardus**
**Irisstraat 41**
**NL-3434 HC Nieuwegein(NL)**
Inventor: **Beuvery, Eduard Coen**
**Kerkstraat 66**
**NL-4132 BG Vianen(NL)**

(74) Representative: **van der Beek, George Frans, Ir. et al**
**Nederlandsch Octrooibureau Scheveningseweg 82 P.O. Box 29720**
**NL-2502 LS 's-Gravenhage(NL)**

EP 0 257 721 B1

## Description

The invention relates to a new vaccine comprising a native protein capable of inducing the production of protective antibodies in a host, together with an adjuvant amount of certain proteins or peptides which, as such, are not capable of inducing substantial protective antibody titres but considerably enhance the immunizing activity of said native proteins.

More specifically the invention relates to a new picornavirus vaccine comprising inactivated picornavirus and certain adjuvant proteins related to picornavirus.

## Background of the invention

It is known to enhance the effectivity of immunizations by administering the immunogen repeatedly. The second and, if necessary, subsequent booster injections induce high titres of protecting antibodies.

It is also known to enhance the effectivity of an immunogen by a pretreatment of the host with a subunit of the native immunogen. This so-called priming effect has been shown, for example, with a peptide comprising part of the amino acid sequence of the B subunit of cholera toxin. This peptide was coupled to a protein carrier, and the conjugate was used for priming immunizations. After priming, injection with a sub-immunizing amount of cholera toxin led to a substantial level of neutralizing antibodies. [Jacob et al., The EMBO Journal 4 (1985), pages 3339-3343].

In case of polio vaccine, Van Wezel et al. [Dev.Biol.Stand. 55 (1984), pages 209-215] have shown a similar priming effect. Injection of poliovirus capsid proteins isolated by means of SDS-PAGE, followed by an injection with inactivated polio vaccine resulted in stimulation of the formation of neutralizing antibodies.

In 1983 Emini et al [Nature 304 (1983), pages 699-703] have described the priming principle in connection with peptides consisting of 5-10 amino acids coupled to carrier proteins. The sequence of the peptides corresponded with parts of the capsid protein I of type 1 poliovirus (strain: Mahoney). In all cases a stimulation of the neutralizing response to type 1 poliovirus was observed.

Viruses of the picornavirus family are RNA containing viruses. Their diameter is about 30 nm and their protein capsid has the form of an icosaeder.

Based on physico-chemical characteristics the family is divided into four genera:
  a. enterovirus (e.g. polio, hepatitis A, echo and coxsackievirus),
  b. cardiovirus (e.g. encephalomyocarditis and Mengovirus),
  c. rhinovirus and
  d. aphtovirus (e.g. foot-and-mouth disease virus).

Within the members of the genera a differentiation into types is made. For example, there are three types (1 to 3) of poliovirus, at least 89 types of rhinovirus and 7 types of foot-and-mouth-diseasevirus.

The poliovirion contains 60 protomes. Each of these protomers consists of four capsid proteins (VP1 to VP4). Capsid proteins VP1, VP2 and VP3 are positioned at the surface of the virion, whereas VP4 is located in the interior of the protein capsid. The position of the three surface-positioned capsid proteins is disclosed in Hogle et al. Science 229 - (1985), pages 1358-65. The three-dimensional structure of these three capsid proteins is essentially identical. In their native conformation the capsid proteins contain four loops and eight so-called beta-sheets.

The amino acid sequence of the capsid proteins of a number of picornaviruses is known. Picornaviruses having a known primary structure are the three types of poliovirus [Nature 291 - (1981), pages 547-553; J.Mol.Biol. 174 (1984), pages 561-585; Proc. Natl. Acad. Sci. USA 81 - (1984), pages 1539-1543], hepatitis A (Proc.Natl. Acad.Sci. USA 82 (1985), pages 2627-2631], two types of rhinovirus [Nucl. Ac. Res. 13 (1985) pages 2111-2126; Proc. Natl. Acad. Sci. USA 82 (1985), pages 732-736], coxsackievirus B3 [Vir. Res. 3 - (1985), pages 263-270], encephalomyocarditisvirus [Nucl.Ac.Res. 12, (1984) pages 2969-2985], and a number of foot-and-mouth disease virus strains [Gene 17 (1982), pages 153-161; Nucl.Ac.Res. 12 - (1984), pages 6587-6601]. Comparison of these sequences shows that the primary structure of the three types of poliovirus is more strongly related to that of the rhinoviruses than to that of heptitis A. Hepatitis A belongs, just as poliovirus, to the genus of enteroviruses. This shows that the relationship within the genus is not necessarily stronger than that outside the genus. Emini et al. (Virology 140 - (1985), pages 13-20) have shown by means of immunochemical techniques that there are relationships between the capsid proteins of a number of enteroviruses and those of rhinovirus 2. Capsid protein 3 showed the strongest cross-reactivity.

Immunization with inactivated polio vaccine (generally formalin inactivated virus suspension of the three types of poliovirus) results in the formation of neutralizing antibodies. These antibodies bind with the intact virus. The position of the antigenic determinants giving rise to the formation of neutralizing antibodies has been determined by means of the so-called "escape mutans" (Nature 301 (1983), pages 674-679; J. Virol. 57 (1986), pages 246-257). It appears in practically all cases that these determinants occur in the loops of the capsid proteins. The capsid proteins of the poliovirus show a strong mutual interaction. As a consequence of this interaction denaturing conditions have to be used for the isolation of the capsid proteins. Injections with the purified capsid proteins of poliovirus does not result in the formation of significant titres of neutralizing antibodies. This lack of capability to induce the formation of neutralizing antibodies has to be ascribed to the fact that the threedimensional structure of the isolated capsid proteins strongly differs from the threedimensional structure of the proteins in the virus.

Toyoda et al. in J.Mol.Biol. 1984, pages 561-585 discloses the primary structure of the capsid proteins of the three types of poliovirus (of each type the Sabin strain was analyzed). Comparison of these structure shows strong sequential homologies for the three types. The positions in the capsid proteins where differences are observed generally are positioned in the loops of the capsid proteins.

At least two lymphoid cell populations are involved in the formation of antibodies after injection of immunogens: The B cell and the helper T cell. The B cell recognizes the antigenic determinant and the helper cell recognizes the carrier determinant after processing of the immunogen. Eventually, the B cell, after differentiation into a plasma cell, is responsible for the formation of antibodies directed to the antigenic determinant. Based on these immunological data, Gupta et al. (Proc.Natl.Acad. Sci. USA 83 (1986), pages 2604-2608) have suggested that the B cell recognizes the antigenic determinant against which neutralizing antibodies are formed, and that the helper T cell recognizes the rest of the virus. As stated above, the rest of the virus contains a large number of common sequences. The recognition by the helper T cell is considerably less conformation-dependent than the recognition by the B cell. Based on this data and the occurrence of common sequences in native immunogens it seems to be probable that T helper cells recognizing these common structures will be formed after immunization with native proteins or after injection with proteins or peptides showing sequential homology with said native proteins.

The stimulation is observed not only with the virus type homologous with the capsid protein (homotypical), but also for the other virus types (heterotypical). The degree of priming stimulation practically corresponds with that of the booster effect when the vaccine is injected twice. The denatured capsid proteins are not or hardly capable of inducing the formation of neutralizing antibodies. It is believed that this priming stimulation obtained by injection with the carrier portion of an immunogen followed by an injection with the complete immunogen results in stimulation of the antibody response to the hapten (antigenic determinant). Most probably, the common primary structures are responsible for the heterotypical stimulation by the capsid proteins.

**Discussion of the Invention**

According to the invention it has been found that immunogenically ineffective proteins and peptids showing sequential homology with a native immunogen do not only show the priming effect when injected some time before vaccination with the native immunogen, but are also useful as adjuvants for said native immunogenic proteins.

The invention is very useful because it provide a vaccine which, by a single vaccination, will induce high titres of neutralizing antibodies with lower doses of native immunogenic protein.

The invention, therefore, provides a vaccine comprising a native protein capable of inducing the production of protective antibodies in a host, together with an adjuvant amount of a protein or peptide showing sequential homology with said native protein but being incapable of effectively inducing the formation of protecting antibodies.

More specifically, the invention provides a picornavirus vaccine comprising as immunogen inactivated picornavirus together with an adjuvant amount of denatured picornavirus capsid protein.

The expression "denatured picornavirus capsid protein" includes proteins isolated from the viral capsid, as well as peptides produced by other methods such as synthetic methods or recombinant DNA techniques insofar as these peptides show sequential homology with the viral capsid proteins. Generally, the denatured picornavirus capsid proteins comprise all of the peptides showing the known priming effect which, as discussed above, is the effect of enhancing the production of neutralizing antibodies by a picornavirus vaccine when the host is injected with the priming peptide some time before the vaccination.

The fact that priming injections or repeated booster vaccinations have now become superfluous is of great advantage to the patient.

It is assumed that the newly found adjuvant effect of the denatured capsid proteins and of the related synthetic peptides is due to an additional stimulation of the T helper cell population involved in the B cell response to the native protein.

Although the invention is illustrated by experimental results obtained with poliovirus, the known presence of identical or similar primary structures in the capsid proteins of viruses as well as the known priming effect occurring with a wide variety of immunogens suggests that the newly found adjuvant effect will be applicable to all kinds of native protein vaccines.

The invention is particularly useful with vaccines comprising inactivated picornavirus, especially inactivated enterovirus, more particularly with enteroviruses selected from the group consisting of inactivated poliovirus, hepatitis A-virus, echovirus, rhinovirus and coxsackievirus.

More specifically, the invention provides vaccines in which the inactivated enterovirus is an inactivated poliovirus selected from the group consisting of poliovirus type 1, poliovirus type 2, poliovirus type 3, and mixtures thereof.

In the vaccines according to the invention it is not necessary for the sequentially homologous proteins or peptides, such as the denatured picornavirus capsid proteins, to be derived from a genus or species identical to that of the native protein used together with the adjuvanting proteins or peptides.

Excellent results have been obtained with a vaccine comprising inactivated poliovirus, such as poliovirus type 1, type 2, type 3 and mixtures thereof, together with denatured poliovirus capsid proteins, viz. VP1, VP2, VP3, VP4 and mixtures thereof. Also in this case, the primary structures of the three types of capsid proteins which are common to all capsid proteins are responsible for the observed heterotypical adjuvant effect upon the neutralizing antibody response to polio vaccine.

The following example is given for illustrative purposes only.

Example

Trivalent inactivated polio vaccine

Trivalent inactivated polio vaccine was prepared as described by Van Wezel et al. (Develop. Biol. Stand., 41, 159-168 (1978), Rev.Inf.Dis., 6, 335-340 (1984)) and contained 10, 1, and 6 D-antigen units per ml for the type 1, 2, 3 vaccine components, respectively.

Preparation of a concentrated viral suspension

Type 1 polio virus (Mahoney strain) was cultivated in a microcarrier culture on tertiary monkey kidney cells. The virus harvest was clarified, concentrated and purified according to the methods described in the references mentioned above. The purified virus suspension was concentrated by the addition of 6.3% (w/v) polyethylene glycol. The opalescent suspension was centrifuged (4,000 x g; 15 min.) and the pellet was resuspended in M 199 and dialyzed against this medium. The virus suspension was clarified (4,000 x g; 15 min.) and the virus was pelleted again (100,000 x g; 4 hr.). The pellet was suspended in RBS buffer (composition: 0.01 M Tris, 0.01 M NaCl and 1.5 mM $MgCl_2$, pH 6.0) and its protein content was determined according to the method of Peterson (Anal. Biochem., 83, 346-356 (1977)).

Isolation of capsid proteins

i. by HPLC

The isolation of the capsid proteins was done essentially as described by Dernick et al. (Virology 130, 243-246 (1983)). Instead of wide pore C18 silica, wide pore phenyl silica from Vydac (The Separation Groups, Hesparia, California, cat. no. 219) was used. A virus sample containing 1.1 mg virus in 160 $\mu$l buffer was mixed with 240 $\mu$l formic acid and incubated during five minutes at room temperature. After clarification (10,000xg;5 min.) a 400 $\mu$l sample was injected and the capsid proteins were eluted in 60% (v/v) formic acid with a linear gradient of 60% formic acid in acetonitrile. Fractions containing the capsid proteins were collected. The excess of formic acid and acetonitrile was removed in a speed vacuum concentrator (Savant Inc., Hickville, NY) in combination with an FTS systems Multi Cool (Stone Ridge, NY). Thereafter, the fractions were dialyzed against 8 M urea and analyzed by SDS-PAGE (Nature 227, 680-685 (1970)). Finally the protein contents of the fractions were assayed.

ii. by SDS-PAGE

The purified virus suspension was incubated in Laemmli sample buffer (Nature 277, 680-685 (1970)) containing 8 M urea, 2% (w/v) SDS and 0.1 M dithiothreitol during three hours at 37°C. Capsid proteins were isolated by preparative SDS-PAGE in a 3 mm thick 10% slab gel containing 1 M urea. The position of the capsid proteins was localized by covering the gel shortly with a sheet of nitrocellulose and subsequently visualizing protein lines on the sheet by an enzyme immune assay as described by Tonbin et al. (Proc.Natl.Acad.Sci.USA 76, 4350-4354 (1979)). The conjugated antiserum was directed to the denatured capsid proteins (Vaccine 1, 17-22 (1983)). The capsid proteins were electro-eluted from the cut-out parts of the gel containing the proteins. Their identity was checked by SDS-PAGE, and assay of Peterson was applied for the determination of their protein contents.

Immunization of rats

Four groups consisting of three home-bred spf Wistar rats were injected intramuscularly with a

mixture of 30 $\mu$g of the separate purified capsid proteins (0.25 ml), trivalent inactivated polio vaccine (injected amount: 2.5, 0.25 and 1.5 D unit for type 1, 2 and 3 components, respectively) and 1.5 mg AlPO$_4$. Five different control groups were injected with the same dose of the four separate purified capsid proteins plus AlPO$_4$ or the same dose of trivalent inactivated polio vaccine plus AlPO$_4$. Blood samples were collected prior and four weeks after injection.

Determination of neutralizing antibodies

The neutralizing antibody titers of the sera were determined in mictrotiter plates by the addition of an infectious polio type 1 (Mahoney strain), type 2 (MEF strain) or type 3 (Saukett strain) virus suspension (100 TCID$_{50}$ for each strain) to a twofold dilution series of the serum samples (50 $\mu$l). The serum-virus mixtures were incubated for 24 hours at 37$^\circ$C. Thereafter 1 x 10$^4$ Hela cells were added and after five days the 50% neutralization titres were determined.

Results immunization experiment

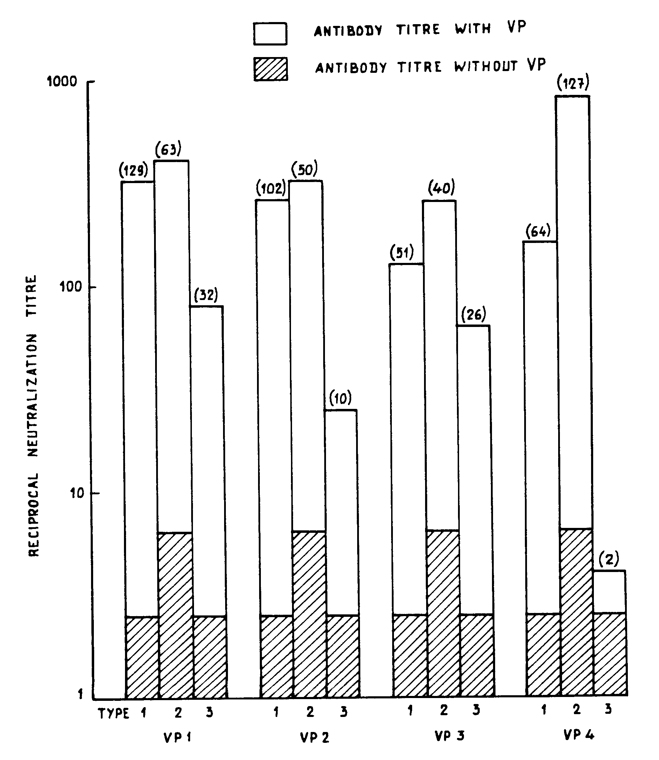

The neutralization titres of serum samples obtained prior and four weeks after immunization were determined in the neutralization assay. Neither pre-immunization serum samples, nor samples obtained from rats injected with the four capsid proteins only, were able to neutralize the three poliovirus strains tested. The antibody titres of the post-immunization samples of the groups injected with the combinations of capsid proteins and trivalent inactivated polio vaccine in comparison with the controls injected with the same low dose of trivalent vaccine only, are shown in the attached drawing. The results are presented as geometric mean titres and indicate that the addition of all four capsid proteins stimulated the neutralization antibody response with a factor of about 10 to more than 100. In the drawing the figures in parenthesis indicate these stimulation factors. The adjuvant effect of the capsid proteins on the type 3 vaccine component was somewhat behind the effect observed for the type 1 and 2 components.

Essentially the same results were observed with the combination of capsid protein 2 isolated by preparative SDS-PAGE and trivalent inactivated polio vaccine.

**Claims**

**1.** A vaccine comprising a native protein capable of inducing the production of protective antibodies in a host, together with an adjuvant amount of a protein or peptide showing sequential homology with said native protein but being incapable of effectively inducing the formation of protecting antibodies.

**2.** The vaccine of claim 1 comprising as immunogen inactivated picornavirus together with an adjuvant amount of denatured picornavirus capsid protein.

**3.** The vaccine of claim 2 in which the inactivated picornavirus is an activated enterovirus.

**4.** The vaccine of claim 3 in which the inactivated enterovirus is selected from inactivated poliovirus, hepatitis A virus, echovirus, rhinovirus and coxsackievirus.

**5.** The vaccine of claim 4 in which the inactivated enterovirus is an inactivated polio virus selected from polio virus type 1, polio virus type 2, polio virus type 3, and mixtures thereof.

**6.** The vaccine of claim 2, in which the denatured picornavirus capsid protein is derived from a picornavirus genus different from that of the inactivated picornavirus present in the vaccine.

**7.** The vaccine of claim 2 in which the denatured picornavirus capsid protein is derived from the same picornavirus genus as that of the inactivated picornavirus present in the vaccine.

**8.** The vaccine of claim 7 in which the inactivated picornavirus is an inactivated enterovirus and the denatured picornavirus capsid protein is a denatured enterovirus capsid protein.

**9.** The vaccine of claim 8 in which the inactivated enterovirus is inactivated poliovirus, and the denatured enterovirus capsid protein is denatured poliovirus capsid protein.

**10.** The vaccine of claim 9 in which the denatured poliovirus capsid protein is selected from VP1, VP2, VP3, VP4, and mixtures thereof.

**11.** The vaccine of claim 9 in which the inactivated poliovirus is selected from inactivated poliovirus type 1, type 2, type 3, and mixtures thereof.

**Revendications**

**1.** Un vaccin comprenant une protéine native capable d'induire la production d'anticorps protecteurs chez un hôte, simultanément à une quantité adjuvante d'une protéine ou peptide présentant une homologie séquentielle avec ladite protéine native mais étant incapable d'induire efficacement la formation d'anticorps protecteurs.

2. Le vaccin de la revendication 1 comprenant comme immunogène du picornavirus inactivé ainsi qu'une quantité adjuvante de protéine de capside de picornavirus dénaturée.

3. Le vaccin de la revendication 2 dans lequel le picornavirus inactivé est un entérovirus inactivé.

4. Le vaccin de la revendication 3 dans lequel l'entérovirus inactivé est sélectionné à partir de virus poliomyélitique, de virus de l'hépatite A, d'échovirus, de rhinovirus et de virus coxsackie inactivés.

5. Le vaccin de la revendication 4 dans lequel l'entérovirus inactivé est un virus poliomyélitique inactivé sélectionné à partir de virus poliomyélitique du type 1, de virus poliomyélitique du type 2, de virus poliomyélitique du type 3 et des mélanges de ceux-ci.

6. Le vaccin de la revendication 2, dans lequel la protéine de capside de picornavirus dénaturée est dérivée d'un genre de picornavirus différent de celui du picornavirus inactivé présent dans le vaccin.

7. Le vaccin de la revendication 2 dans lequel la protéine de capside de picornavirus dénaturée est dérivée du même genre de picornavirus que celui du picornavirus inactivé présent dans le vaccin.

8. Le vaccin de la revendication 7 dans lequel le picornavirus inactivé est un entérovirus inactivé et la protéine de capside de picornavirus dénaturée est une protéine de capside d'entérovirus dénaturée.

9. Le vaccin de la revendication 8 dans lequel l'entérovirus inactivé est le virus poliomyélitique inactivé, et la protéine de capside d'entérovirus dénaturée est la protéine de capside de virus poliomyélitique dénaturée.

10. Le vaccin de la revendication 9 dans lequel la protéine de capside de virus poliomyélitique dénaturée est sélectionnée à partir des protéines VP1, VP2, VP3, VP4, et de mélanges de celles-ci.

11. Le vaccin de la revendication 9 dans lequel le virus poliomyélitique inactivé est sélectionné à partir de virus poliomyélitique inactivé de type 1, de type 2, de type 3, et de mélanges de ceux-ci.

**Patentansprüche**

1. Vakzin mit einem nativen Protein, das fähig ist, die Erzeugung schützender Antikörper in einem Gastkörper herbeizuführen, zusammen mit einer adjuvierenden Menge eines Proteins oder Peptids, welches sequentielle Homologie mit dem nativen Protein zeigt, jedoch nicht fähig ist, wirksam die Bildung schützender Antikörper herbeizuführen.

2. Vakzin nach Anspruch 1, welches als Immunogen inaktiviertes Picornavirus mit einer adjuvierenden Menge denaturiertem Picornaviruscapsid-Protein umfasst.

3. Vakzin nach Anspruch 2, bei dem das inaktivierte Picornavirus ein inaktiviertes Enterovirus ist.

4. Vakzin nach Anspruch 3, bei dem das inaktivierte Enterovirus aus inaktiviertem Poliovirus, Hepatitis-A-Virus, ECHO-Virus, Rhinovirus und Coxsackievirus ausgewählt ist.

5. Vakzin nach Anspruch 4, bei dem das inaktivierte Enterovirus ein inaktiviertes Poliovirus ist, das aus Poliovirus des Typs 1, Poliovirus des Typs 2, Poliovirus des Typs 3 und Mischungen davon ausgewählt ist.

6. Vakzin nach Anspruch 2, bei dem das denaturierte Picornaviruscapsid-Protein von einer Picornavirus-Gattung abgeleitet ist, die von der des im dem Vakzin vorhandenen inaktivierten Picornavirus verschieden ist.

7. Vakzin nach Anspruch 3, bei dem das denaturierte Picornaviruscapsid-Protein von derselben Picornavirus-Gattung abgeleitet ist wie die des in dem Vakzin vorhandenen inaktivierten Picornavirus.

8. Vakzin nach Anspruch 7, bei dem das inaktivierte Picornavirus ein inaktiviertes Enterovirus und das denaturierte Picornaviruscapsid-Protein ein denaturiertes Enteroviruscapsid-Protein ist.

9. Vakzin nach Anspruch 8, bei dem das inaktivierte Enterovirus inaktiviertes Poliovirus und das denaturierte Enteroviruscapsid-Protein denaturiertes Polioviruscapsid-Protein ist.

**10.** Vakzin nach Anspruch 9, bei dem das denaturierte Polioviruscapsid-Protein aus VP1, VP2, VP3, VP4 und Mischungen davon ausgewählt ist.

**11.** Vakzin nach Anspruch 9, bei dem das inaktivierte Poliovirus aus inaktiviertem Poliovirus des Typs 1, des Typs 2, des Typs 3 und Mischungen davon ausgewählt ist.